# DEMANDE DE BREVET EUROPEEN

(11) **EP 1 800 602 A1**
(43) Date de publication de la demande: **27.06.2007**
(21) Numéro de dépôt: 06025772.2
(22) Date de dépôt: 13.12.2006
(51) Int. Cl.: A61B 5/18, A61B 5/11, B60K 28/06, B60N 2/00

(54) **Surveillance de vigilance d'un conducteur de véhicule basée sur les mouvements du conducteur**

(30) Priorité: 23.12.2005 FR 0513249
(71) Demandeur: Siemens VDO Automotive, 31036 Toulouse (FR)
(72) Inventeur: Boverie, Serge, 31830 Plaisance du Touch (FR)

(57) **Abrégé**

L'invention concerne un procédé de détection de mouvements caractéristiques d'un conducteur de véhicule en vue d'un diagnostic de baisse de vigilance du dit conducteur. Selon l'invention, et dans une phase préalable, on divise la surface de l'assise (A) et du dossier (D) du siège (S) en une pluralité de surfaces élémentaires dans chacune desquelles on implante un capteur de pression (An,n - Dn,n) apte à détecter les appuis et les relâchements exercés par le conducteur sur le dit capteur de pression, et on sélectionne une pluralité de mouvements spécifiques que l'on caractérise, chacun, par la détermination d'au moins une zone géographique soumise à un « appui » ou un « relâchement » résultant du dit mouvement. De plus, le traitement des données consiste à analyser les signaux de mesure délivrés par les capteurs de pression, et à comparer, à chaque instant d'échantillonnage t, les éventuelles zones géographiques d'appui et de relâchement décelées avec chacune des zones géographiques mémorisées, de façon fournir une information de détection d'un mouvement spécifique lors d'une corrélation entre les dites zones géographiques.

## Description

L'invention concerne un procédé de détection de mouvements caractéristiques d'un conducteur de véhicule en vue d'un diagnostic de baisse de vigilance du dit conducteur.

Parmi les informations représentatives de l'évolution de la baisse de vigilance d'un conducteur, il a été établi que certains mouvements du corps des conducteurs peuvent constituer un bon indicateur en vue de l'élaboration d'un diagnostic de baisse de vigilance.

Sur la base de ce principe, l'objectif essentiel de l'invention est de fournir un procédé permettant de détecter de façon systématique et fiable des mouvements spécifiques prédéterminés du corps d'un conducteur.

A cet effet, et en premier lieu, l'invention vise un procédé de détection de mouvements caractéristiques d'un conducteur de véhicule en position de conduite sur un siège comportant une assise et un dossier, le dit procédé de détection consistant :
- à diviser la surface de l'assise et du dossier du siège en une pluralité de surfaces élémentaires dans chacune desquelles on implante un capteur de pression apte à détecter :
   - une augmentation de la pression exercée sur la surface élémentaire, représentative d'une pression positive, dite « appui » exercée par le conducteur sur le capteur de pression,
   - une diminution de la pression exercée sur la surface élémentaire, représentative d'une diminution ou d'une annulation, dite « relâchement », de la pression exercée par le conducteur sur le capteur de pression,
- à sélectionner une pluralité de mouvements spécifiques, tels que mouvements du haut du corps, repositionnement sur l'assise (A), basculement transversal vers une position de conduite décalée sur un côté, basculement longitudinal vers une position de conduite enfoncée dans le haut du dossier (D), et à caractériser chacun des dits mouvements spécifiques par la détermination d'au moins une zone géographique soumise à un « appui » ou un « relâchement » résultant du dit mouvement,
- et à analyser, avec une période d'échantillonnage donnée, les signaux de mesure délivrés par les capteurs de pression, et à comparer, à chaque instant d'échantillonnage t, les éventuelles zones géographiques d'appui et de relâchement décelées avec chacune des zones géographiques prédéterminées, de façon à fournir une information de détection d'un mouvement spécifique lors d'une corrélation entre les dites zones géographiques.

De façon avantageuse, selon l'invention, les zones géographiques caractéristiques des mouvements spécifiques correspondent à des zones enveloppes des surfaces de contact avec l'assise et le dossier de différentes parties du corps du conducteur : dos complet, parties du dos (gauche, droite, haute, basse), assise complète ou partie basse ou haute de l'assise, postérieur ou parties de postérieur (droite ou gauche), jambe gauche ou droite...

De plus, ce procédé consiste, avantageusement, en vue de la caractérisation de mouvements spécifiques donnés, à déterminer au moins une zone géographique soumise à un « appui » ou un « relâchement », ainsi qu'une séquence temporelle de sollicitation de la dite zone géographique consistant en une alternance d'une période d'activation, par appui ou relâchement, et d'une période de non activation.

Un tel procédé défini ci-dessus de façon générale permet de détecter divers types de mouvements et particulièrement quatre mouvements spécifiques avantageux détaillés ci-après.

Le premier de ces mouvements consiste en un mouvement du haut du dos, et en vue de sa détection et de façon avantageuse selon l'invention :
- on détermine, en vue de la caractérisation du dit mouvement :
   - au moins une zone géographique soumise à un « appui », correspondant à une zone enveloppe des surfaces de contact avec le dossier de la portion centrale du dos d'un conducteur,
   - et au moins une zone géographique soumise à un « relâchement », correspondant à une zone enveloppe des surfaces de contact avec l'assise du fessier d'un conducteur,
- et on délivre une information de détection d'un mouvement du haut du dos, uniquement lors de la corrélation d'une zone géographique décelée avec :
   - une zone géographique précitée du dossier,
   - et / ou une zone géographique précitée de l'assise, moyennant alors, en outre, un degré minimal de symétrie, par rapport à un axe longitudinal de l'assise, des surfaces élémentaires constituant la dite zone géographique décelée.

Le second mouvement consiste en un mouvement de repositionnement, et en vue de sa détection et de façon avantageuse selon l'invention :
- on détermine deux zones géographiques soumises à un « relâchement », correspondant chacune à une zone enveloppe de la surface de contact d'une jambe du conducteur avec l'assise,
- et on délivre une information de détection d'un mouvement de repositionnement, uniquement lors de la détection de la séquence temporelle suivante :
   - à un instant t : corrélation d'une zone géographique décelée avec une des zones géographiques précitées de l'assise,
   - et à un instant t+1 : absence d'activation de la zone géographique corrélée à l'instant t, combinée avec une corrélation d'une zone géographique décelée avec l'autre zone géographique de l'assise.

Deux autres procédures peuvent également être mises en oeuvre selon l'invention pour détecter un mouvement de repositionnement.

Selon la première de ces deux procédures, et de façon avantageuse selon l'invention :
- on détermine, en vue de la caractérisation du mouvement :
   - deux zones géographiques soumises à un « appui », correspondant aux deux zones enveloppes respectives des surfaces de contact avec le dossier des parties droite et gauche du bas du dos d'un conducteur,
   - et deux zones géographiques soumises à un « relâchement », correspondant aux deux zones enveloppes respectives des surfaces de contact avec l'assise des parties droite et gauche du postérieur d'un conducteur,
- et on délivre une information de détection d'un mouvement de repositionnement, lors, simultanément, de :
   - la corrélation d'une zone géographique décelée avec une zone géographique précitée droite (ou gauche), du dossier, combinée avec l'absence d'activation de l'autre zone géographique précitée du dossier,
   - la corrélation d'une zone géographique décelée avec la zone géographique précitée gauche (ou droite) de l'assise, combinée avec l'absence d'activation de l'autre zone géographique précitée de l'assise.

Selon la seconde procédure, et de façon avantageuse selon l'invention :
- on détermine, en vue de la caractérisation du mouvement de repositionnement :
   - une zone géographique soumise à un « appui », correspondant à la zone enveloppe de la surface de contact avec le dossier du dos complet d'un conducteur,
   - et une zone géographique soumise à un « relâchement », correspondant à la zone enveloppe de la surface de contact avec l'assise de l'assise complète d'un conducteur,
- et on délivre une information de détection d'un mouvement de repositionnement lors de la corrélation d'une zone géographique décelée avec la zone géographique précitée du dossier et / ou la zone géographique précitée de l'assise.

Le troisième mouvement spécifique visé par l'invention consiste en un mouvement de transition vers une position de conduite décalée, et en vue de sa détection et de façon avantageuse selon l'invention :
- on détermine, en vue de la caractérisation du dit mouvement :
   - deux zones géographiques soumises à un « appui », correspondant aux deux zones enveloppes respectives des surfaces de contact avec le dossier des parties droite et gauche du dos d'un conducteur,
   - et deux zones géographiques soumises à un « relâchement », correspondant chacune à une zone enveloppe de la surface de contact d'une jambe du conducteur avec l'assise,
- et on délivre une information de détection d'un mouvement de transition, uniquement lors de la détection de la séquence temporelle suivante :
   - à un instant t : corrélation d'une zone géographique décelée avec une des zones géographiques précitées de l'assise, et absence d'activation de la zone géographique du dossier symétrique de la dite zone décelée de l'assise,
   - à un instant t+1 : absence d'activation de la zone géographique de l'assise corrélée à l'instant t, combinée avec une corrélation d'une zone géographique décelée avec l'autre zone géographique de l'assise.

Le quatrième mouvement spécifique visé par l'invention consiste, enfin, en un mouvement de transition vers une position de conduite « enfoncée » dans le haut du dossier. En vue de la détection de ce mouvement ; et de façon avantageuse selon l'invention :
- on détermine, en vue de la caractérisation du dit mouvement :
   - deux zones géographiques soumises à un « appui », correspondant aux deux zones enveloppes respectives des surfaces de contact avec le dossier des parties haute et basse du dos d'un conducteur,
   - et deux zones géographiques soumises à un « relâchement », correspondant aux deux zones enveloppes respectives des surfaces de contact avec l'assise des parties haute et basse de l'assise d'un conducteur,
- et on délivre une information de détection d'un mouvement de transition lors, d'une part, de la corrélation simultanée de deux zones géographiques décelées avec respectivement les zones géographiques précitées correspondant aux parties hautes du dos et de l'assise, et d'autre part, de l'absence combinée d'activation des zones géographiques précitées correspondant aux parties basses du dos et de l'assise.

Par ailleurs, on utilise avantageusement, selon l'invention, des capteurs de pression consistant en des capteurs de pression différentiels aptes à mesurer la dynamique de la force appliquée sur la surface élémentaire correspondante.

Par ailleurs , lors de la mise en oeuvre du procédé selon l'invention, on procède avantageusement; durant une phase préalable suivant le démarrage du véhicule, dite phase de seuillage, à un traitement des signaux délivrés par les capteurs de pression, consistant à calculer, pour chacun des dits capteurs, les moyennes respectives des plus grandes variations positives et négatives des valeurs des dits signaux, de façon à déterminer des valeurs de seuils respectivement négatif et positif pour l'extraction ultérieure des signaux représentatifs d'un appui ou d'un relâchement.

Une telle phase de seuillage permet d'adapter les seuils utilisés par la suite pour l'extraction des signaux « utiles » en fonction du comportement de chaque conducteur.

Pour ce faire, ces seuils adaptatifs sont, en outre, calculés durant les premières minutes de conduite, c'est-à-dire durant une période où, normalement, le conducteur ne se trouve pas dans un état d'hypovigilance, et où il conduit de façon stable.

Par ailleurs, en vue d'affiner et de compléter l'information transmise :
- on attribue à chaque surface élémentaire activée d'une zone géographique, un poids pondéré par la mise en oeuvre d'une méthode mathématique telle que la logique floue, en fonction :
   - de la position géographique, notamment positionnement en zone limitrophe, de la dite surface élémentaire à l'intérieur de la zone géographique,
   - et / ou de l'amplitude du signal fourni par le capteur de pression associé, représentative du niveau d'activation de la dite surface élémentaire,
- et on détermine, pour chaque zone géographique, un degré d'activation de cette dernière calculé à partir du poids de chacune de ses surfaces élémentaires, de façon à associer à chaque information de détection d'un mouvement spécifique, une information représentative du degré d'appartenance à cette catégorie de mouvement.

D'autres caractéristiques buts et avantages de l'invention ressortiront de la description détaillée qui suit en référence aux dessins annexés qui en représentent à titre d'exemple non limitatif un mode de réalisation préférentiel. Sur ces dessins :
- La figure 1 est une vue en perspective schématique d'un siège de véhicule automobile destiné à la mise en oeuvre du procédé de détection selon l'invention,
- la figure 2 est une vue schématique illustrant le type et le principe de capteur de pression utilisé selon l'invention,
- les figures 3 et 4 sont deux graphiques illustrant le procédé de traitement selon l'invention des signaux de mesure délivrés par les capteurs de pression,
- la figure 5 est une vue de face schématique d'un siège de véhicule automobile illustrant une étape du procédé de détection d'un mouvement du haut du dos,
- les figures 6a et 6b sont deux vues de face schématiques d'un siège de véhicule automobile illustrant une étape du procédé de détection d'un mouvement de transition vers une position de conduite décalée vers le côté,
- la figure 7 est une vue de face schématique d'un siège de véhicule automobile illustrant une étape du procédé de détection d'un mouvement de transition vers une position de conduite « enfoncée » dans le haut du dossier,
- et les figures 8a-8b, 8c-8d, et 8e-8f constituent trois groupes de figures, chacun composé de deux vues de face schématiques d'un siège de véhicule automobile illustrant une étape d'un procédé de détection d'un mouvement de repositionnement.

Le procédé selon l'invention décrit ci-dessous en référence aux dessins annexés consiste en un procédé de détection de quatre catégories différentes de mouvements spécifiques effectués par un conducteur de véhicule en position de conduite sur un siège S comportant une assise A et un dossier D. De façon plus spécifique, ce procédé de détection vise à permettre d'élaborer un diagnostic de baisse de vigilance du dit conducteur.

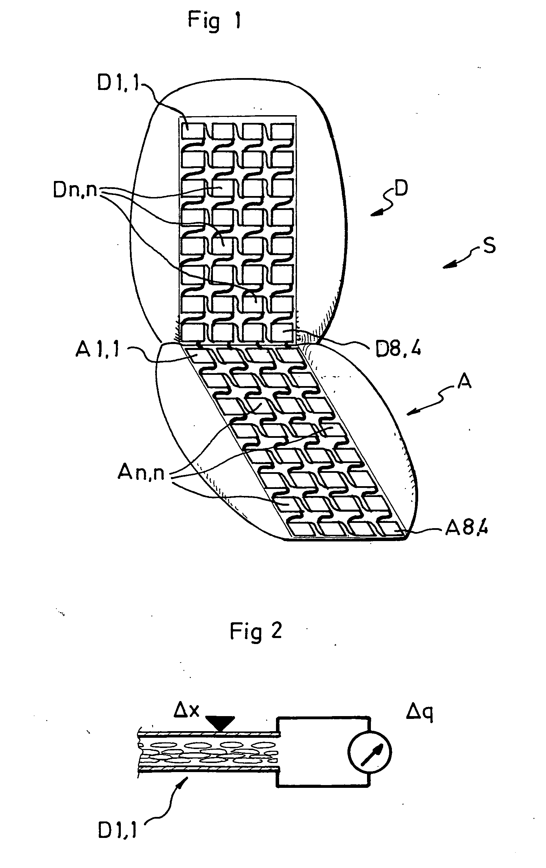

Tel que représenté à la figure 1, en vue de la mise en oeuvre de ce procédé de détection, l'assise A et le dossier D du siège S sont revêtus chacun d'une nappe de capteurs de pression souples, respectivement An,n et Dn,n, délimitant chacun une surface élémentaire d'assise ou de dossier.

En l'exemple, chaque nappe de capteurs est constituée d'une matrice de trente deux capteurs répartis selon 8 lignes et 4 colonnes orthogonales, à savoir respectivement :
- 32 capteurs A1,1 à A8,4 répartis sur l'assise A de façon à fournir des signaux représentatifs des mouvements du bas du cops,
- et 32 capteurs D1,1 à D8,4 répartis sur le dossier D de façon à fournir des signaux représentatifs des mouvements du haut du corps.

De plus, tel que représenté à la figure 2, chaque capteur de pression tel que D1,1 est du type constitué d'un matériau poreux isolant composé de minces couches de polymères séparées par des poches d'air.

Un tel matériau possède comme particularité de renfermer des charges électriques microscopiques et de présenter un comportement similaire à celui des matériaux piézo-électriques.

Tel que représenté à la figure 2, ce comportement de type piézo-électrique se traduit par la génération d'une charge électrique Δq lors de l'application d'une force Δx tendant à faire varier l'épaisseur du matériau poreux constituant le capteur de pression.

Ainsi, une force d'appui appliquée sur un capteur de pression se traduit par un pic positif au niveau du signal fourni par le dit capteur de pression, correspondant à la compression du matériau poreux, tandis qu'un pic négatif est généré lorsque la pression est relâchée.

Par conséquent, de tels capteurs de pression offrent l'avantage de pouvoir détecter des variations d'appui du fait qu'ils mesurent la dynamique de la force appliquée.

Selon l'invention, le traitement du signal fourni par chaque capteur de pression consiste, en premier lieu, à filtrer ce signal de façon à éliminer les bruits parasites engendrés par la route, les vibrations de la voiture...qui se situent dans la partie haute du spectre de fréquences du signal.

A cet effet, ce filtrage est réalisé par un « moyenneur ».

La deuxième opération de traitement des signaux fournis par les capteurs de pression consiste en une opération de seuillage mise en oeuvre uniquement durant une phase préalable suivant le démarrage du véhicule, dite phase de seuillage, au cours de laquelle on réalise un traitement des signaux consistant à calculer, pour chacun des dits capteurs, les moyennes respectives des plus grandes variations positives et négatives des valeurs des dits signaux, de façon à déterminer des valeurs de seuils respectivement négatif et positif pour l'extraction ultérieure des signaux.

En premier lieu, ce traitement est mis en oeuvre lors de chaque démarrage du véhicule, durant les premières minutes suivant ce démarrage, de sorte que les seuils sont adaptés aux caractéristiques de conduite du conducteur présent dans le véhicule.

De plus, ce traitement conduit à calculer un seuil positif et un seuil négatif, de façon à prendre en compte le comportement légèrement dissymétrique des capteurs de pression, et à compenser les différences de dynamique découlant de ce comportement dissymétrique.

Enfin, ce traitement s'applique également de façon séparée respectivement aux capteurs An,n intégrés dans l'assise A et aux capteurs Dn,n intégrés dans le dossier D, de façon à prendre en compte le fait que le poids du corps du conducteur engendre des variations d'amplitude beaucoup plus importantes au niveau de l'assise par rapport à celles que ce poids engendre au niveau du dossier.

En conclusion, ce traitement conduit donc à déterminer quatre seuils consistant en un seuil moyen positif et un seuil moyen négatif pour chacune des surfaces, assise A et dossier D.

La dernière opération de traitement appliquée aux signaux fournis par les capteurs de pression intervient une fois la phase de seuillage terminée, et cette opération a pour objectif de détecter les instants où les valeurs des dits signaux dépassent les valeurs des seuils déterminés lors de la dite phase de seuillage.

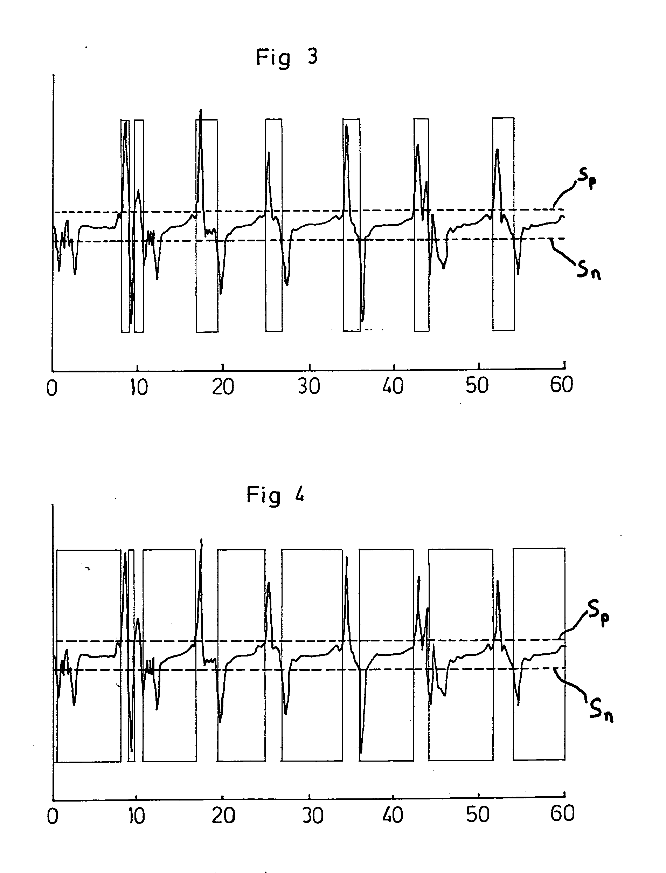

Tel que représenté aux figures 3 et 4 sur lesquelles sont également matérialisés un seuil moyen positif Sp et un seuil moyen négatif Sn, ce traitement conduit à extraire :
- les séquences, dites d'appui, représentées à la figure 3, activées par le dépassement d'un seuil positif et désactivées par le dépassement d'un seuil négatif, et qui correspondent, chacune, à une période de temps durant laquelle le conducteur exerce une force de pression sur le capteur de pression, qui prend fin lors du relâchement de cette pression,
- et les séquences, dites de relâchement, représentées à la figure 4, activées par le dépassement d'un seuil négatif et désactivées par le dépassement d'un seuil positif, et qui correspondent, chacune, à une période de temps durant laquelle le conducteur « supprime » la force de pression exercée sur le capteur de pression, par exemple du fait de l'absence de contact avec le dit capteur, la dite période prenant fin lors d'un nouvel appui exercé sur le capteur.

Le procédé de détection selon l'invention nécessite par ailleurs, dans une phase préalable, de caractériser, essentiellement par des analyses expérimentales, des mouvements spécifiques susceptibles de permettre d'élaborer un diagnostic de baisse de vigilance, la dite caractérisation consistant, dans sa généralité :
- soit à déterminer une ou plusieurs zone(s) géographique(s) soumise(s) à un « appui » ou un « relâchement » du fait de l'exécution du dit mouvement,
- soit à déterminer au moins une zone géographique soumise à un « appui » ou un « relâchement », ainsi qu'une séquence temporelle de sollicitation de la dite zone géographique consistant en une alternance d'une période d'activation, par appui ou relâchement, et d'une période de non activation,

Ce procédé de détection consiste, en outre, à analyser, à chaque instant, les zones d'appui et de relâchement décelées par agrégation des données élémentaires issues des capteurs de pression, et de comparer ces zones décelées avec les zones géographiques caractéristiques prédéfinies.

A titre d'exemple, quatre catégories de mouvements sont définies et explicitées ci-après :
- les mouvements du haut du corps ; exemple : dos qui se décolle du dossier D,
- les mouvements vers une position de conduite décalée sur un côté; exemple : position d'appui contre la vitre ou sur l'accoudoir,
- les mouvements vers une position de conduite « enfoncée » dans le haut du dossier D ; exemple : position de relaxation du conducteur lorsque ce dernier prend appui sur le volant,
- et les mouvements de repositionnement sur le siège; exemple : changement de la zone d'appui sur l'assise A ou sur le dossier D.

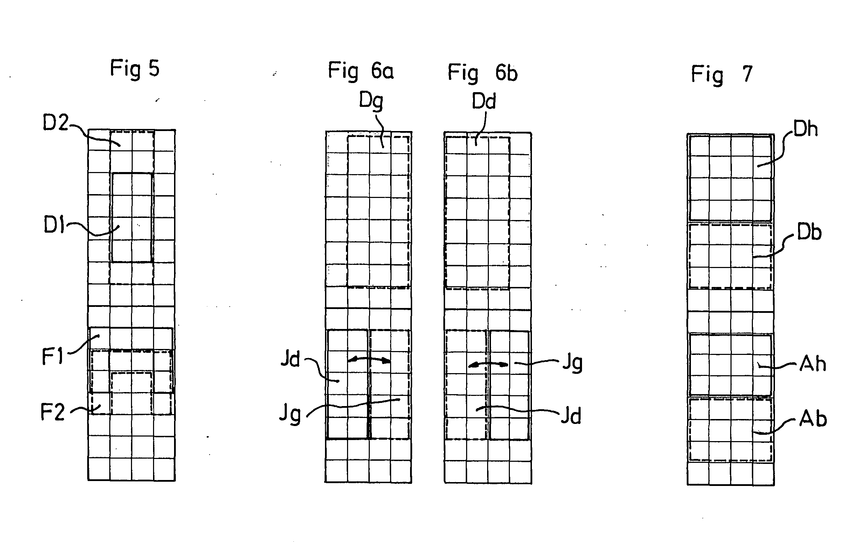

(II est à noter que sur les figures 5 à 8, les zones géographiques délimitées par des traits pleins correspondent à des zones dont les surfaces élémentaires doivent être activées, par un appui ou un relâchement, pour être considérées. Au contraire, les zones géographiques délimitées par des traits pointillés correspondent à des zones dont les surfaces élémentaires ne doivent pas être activées pour être considérées.)

En premier lieu, le premier mouvement, relatif à un mouvement du haut du dos, se caractérise par quatre zones géographiques de localisation représentées à la figure 5.

Tel que représenté à la figure 5, deux de ces zones géographiques D1 et D2 sont situées au niveau du dossier D, et définissent deux surfaces qui se recoupent, centrées sur l'axe de symétrie longitudinal du dossier D.

Les deux autres zones F1 et F2 sont situées au niveau de l'assise A et définissent deux surfaces de même forme et superficie correspondant à la surface de contact avec la dite assise, d'un fessier. De plus, ces deux zones F1, F2 sont décalées longitudinalement l'une par rapport à l'autre, de façon à présenter toutefois une portion de recoupement.

Une telle configuration des zones de localisation des mouvements du haut du dos permet une détection adaptée aux différentes postures de conduite d'un individu.

De plus, lors de leur activation, les zones de localisation D1 et D2 consistent en des zones de relâchement tandis que les deux autres zones F1 et F2 consistent en des zones d'appui. En effet, les mouvements du haut du dos se caractérisent par un décollement du dos par rapport au dossier D, et par une application d'une force d'appui au niveau de l'assise A.

Sur la base de ces définitions des zones de localisation, un mouvement décelé est déterminé comme constituant un mouvement du haut du dos si au moins une des règles suivantes est respectée :
- zone de localisation D1 activée,
- zone de localisation D2 activée,
- zone de localisation F1 activée avec un degré minimal de symétrie, par rapport à un axe longitudinal de l'assise (A), des surfaces élémentaires constituant la dite zone de localisation,
- zone de localisation F2 activée avec un degré minimal de symétrie, par rapport à un axe longitudinal de l'assise (A), des surfaces élémentaires constituant la dite zone de localisation.

En second lieu, le deuxième mouvement relatif à un mouvement vers une position de conduite décalée sur un côté se caractérise, par quatre zones géographiques de localisation représentées aux figures 6a et 6b.

Deux de ces zones géographiques Dd, Dg sont situées au niveau du dossier D et correspondent respectivement à des surfaces de contact avec le dit dossier des parties droite et gauche du dos d'un conducteur.

Les deux autres zones géographiques Jd, Jg sont situées au niveau de l'assise A et correspondant respectivement à des surfaces de contact des jambes gauche et droite du conducteur avec la dite assise.

De plus, lors de leur activation, les zones de localisation Dd et Dg consistent en des zones d'appui tandis que les deux autres zones Jd et Jg consistent en des zones de relâchement.

En outre, la caractérisation de ce mouvement comporte également une composante temporelle définie par les règles énoncées ci-après, consistant à étudier l'activation par relâchement d'une jambe puis l'activation par relâchement de l'autre jambe.

Sur la base de cette composante temporelle et des définitions des zones de localisation, un mouvement décelé est déterminé comme constituant un mouvement vers une position de conduite décalée sur le côté si au moins une des règles suivantes est respectée :
- zone de localisation Jg activée, zone de localisation Jd non activée, alors que cette zone de localisation Jd était précédemment activée, et zone de localisation Dd non activée,
- zone de localisation Jg non activée, zone de localisation Jd activée, alors que la zone de localisation Jg était précédemment activée, et zone de localisation Dg non activée.

Le troisième mouvement relatif à un mouvement de transition vers une position « enfoncée » dans le dossier D, se caractérise, quant à lui, par quatre zones géographiques de localisation représentées à la figure 7.

Deux de ces zones géographiques Dh, Db sont situées au niveau du dossier D et correspondent respectivement à des surfaces de contact avec le dit dossier des parties haute et basse du dos d'un conducteur,

Les deux autres zones géographiques Ah et Ab sont situées au niveau de l'assise A et correspondent respectivement à des surfaces de contact avec l'assise A des parties haute et basse de l'assise du conducteur.

De plus, lors de leur activation, les zones de localisation Dh et Db consistent en des zones d'appui, tandis que les deux autres zones Ah et Ab consistent en des zones de relâchement.

Sur la base de ces définitions des zones de localisation, un mouvement décelé est déterminé comme constituant un mouvement de transition vers une position « enfoncée » dans le dossier D si la règle suivante est respectée :
- zone de localisation Dh activée et zone de localisation Db non activée, zone de localisation Ah activée et zone de localisation Ab non activée.

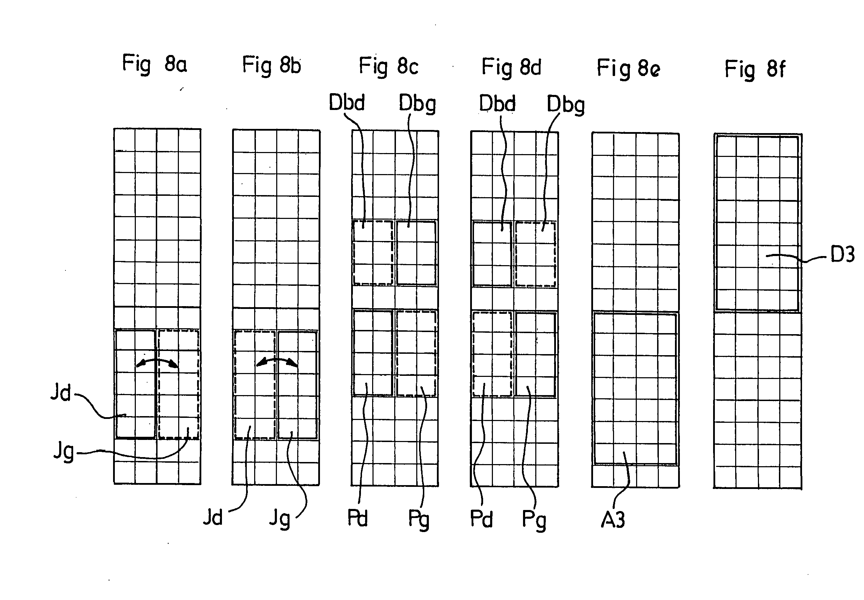

Enfin trois procédures différentes de caractérisation respectivement représentées aux figures 8a-8b, 8c-8d et 8e-8f permettent d'identifier un mouvement de repositionnement.

Pour chacune de ces procédures, en outre, lors de leur activation, les zones de localisation situées au niveau du dossier D consistent en des zones d'appui, tandis que les zones de localisation situées au niveau de l'assise A consistent en des zones de relâchement.

La première procédure s'apparente à celle décrite en référence aux figures 6a et 6b, et selon cette procédure, le mouvement de repositionnement (d'une jambe sur l'autre) se caractérise par:
- deux zones géographiques de localisation Jd et Jg, représentées aux figures 8a et 8b, situées au niveau de l'assise A et correspondant respectivement à des surfaces de contact des jambes gauche et droite du conducteur avec la dite assise,
- une composante temporelle définie par les règles énoncées ci-après, consistant à étudier l'activation par relâchement d'une jambe puis l'activation par relâchement de l'autre jambe.

Selon cette procédure, un mouvement décelé est déterminé comme constituant un mouvement de repositionnement si au moins une des règles suivantes est respectée :
- zone de localisation Jg activée, zone de localisation Jd non activée, alors que cette zone de localisation Jd était précédemment activée,
- zone de localisation Jg non activée, zone de localisation Jd activée, alors que la zone de localisation Jg était précédemment activée.

Selon la deuxième procédure, un mouvement de repositionnement se caractérise par quatre zones géographiques de localisation représentées aux figures 8c et 8d.

Deux de ces zones géographiques Dbd, Dbg sont situées au niveau du dossier D et correspondent respectivement à des surfaces de contact avec le dit dossier des parties droite et gauche du bas du dos d'un conducteur,

Les deux autres zones géographiques Pd, Pg sont situées au niveau de l'assise A et correspondent respectivement à des surfaces de contact avec la dite assise des parties gauche et droite du postérieur du conducteur.

Selon cette deuxième procédure, un mouvement décelé est déterminé comme constituant un mouvement de repositionnement si la règle suivante est respectée :
- zone de localisation Dbd non activée et zone de localisation Dbg activée, et zone de localisation Pd activée et zone de localisation Pg non activée.

Enfin, selon la troisième procédure, un mouvement de repositionnement se caractérise par deux zones géographiques de localisation consistant en :
- une zone de localisation A3 représentée à la figure 8e, correspondant à une surface de contact avec l'assise A de l'assise complète d'un conducteur,
- et une zone de localisation D3 représentée à la figure 8f, correspondant à une surface de contact avec le dossier D du dos complet d'un conducteur.

Selon cette troisième procédure, un mouvement décelé est déterminé comme constituant un mouvement de repositionnement si au moins une des règles suivantes est respectée :
- zone de localisation A3 activée,
- zone de localisation D3 activée

Par ailleurs, le procédé selon l'invention ci-dessus décrit peut avantageusement faire appel à des notions de logique floue, par exemple en attribuant à chaque surface élémentaire activée d'une zone géographique, un poids pondéré, par la mise en oeuvre de règles de logique floue, en fonction :
- de la position géographique, notamment positionnement en zone limitrophe, de la dite surface élémentaire à l'intérieur de la zone géographique,
- et / ou de l'amplitude du signal fourni par le capteur de pression associé, représentative du niveau d'activation de la dite surface élémentaire.

De plus, dans ce cas, on détermine, pour chaque zone géographique, un degré d'activation de cette dernière calculé à partir du poids de chacune de ses surfaces élémentaires, de façon à associer à chaque information de détection d'un mouvement spécifique, une information représentative du degré d'appartenance à cette catégorie de mouvement.

En dernier lieu, une analyse finale de cohérence permet de finaliser la détection des mouvements spécifiques et de fournir des résultats plus robustes. Cette analyse de cohérence consiste à réaliser, au fur et à mesure du traitement des données, une expertise en ligne des résultats issus de la classification des mouvements spécifiques.

De façon plus spécifique, une telle analyse de cohérence consiste, au terme de fenêtres glissantes de traitement d'une durée classique donnée, d'une part, à éliminer les informations isolées (artefacts), et d'autre part, à agréger les séquences disjointes relatives à une information identique.

## Revendications

1. Procédé de détection de mouvements caractéristiques d'un conducteur de véhicule en position de conduite sur un siège (S) comportant une assise (A) et un dossier (D), en vue d'un diagnostic de baisse de vigilance du dit conducteur, le dit procédé de détection comportant les étapes suivantes :
• division de la surface de l'assise (A) et du dossier (D) du siège (S) en une pluralité de surfaces élémentaires dans chacune desquelles on implante un capteur de pression (An,n - Dn,n) apte à détecter :
- une augmentation de la pression exercée sur la surface élémentaire, représentative d'une pression positive, dite « appui » exercée par le conducteur sur le capteur de pression,
- une diminution de la pression exercée sur la surface élémentaire, représentative d'une diminution ou d'une annulation, dite « relâchement », de la pression exercée par le conducteur sur le capteur de pression,
• sélection d'une pluralité de mouvements spécifiques, tels que mouvements du haut du corps, repositionnement sur l'assise (A), basculement transversal vers une position de conduite décalée sur un côté, basculement longitudinal vers une position de conduite enfoncée dans le haut du dossier (D), et caractérisation de chacun des dits mouvements spécifiques par la détermination d'au moins une zone géographique soumise à un « appui » ou un « relâchement » résultant du dit mouvement,
• et analyse, avec une période d'échantillonnage donnée, des signaux de mesure délivrés par les capteurs de pression (An,n - Dn,n), et comparaison, à chaque instant d'échantillonnage t, les éventuelles zones géographiques d'appui et de relâchement décelées avec chacune des zones géographiques prédéterminées, de façon à fournir une information de détection d'un mouvement spécifique lors d'une corrélation entre les dites zones géographiques, les dits mouvements spécifiques correspondant à des zones enveloppes des surfaces de contact avec l'assise (A) et le dossier (D) de différentes parties du corps du conducteur : dos complet, parties du dos (gauche, droite, haute, basse), assise complète ou partie basse ou haute de l'assise, postérieur ou parties de postérieur (droite ou gauche), jambe gauche ou droite...,
le dit procédé étant **caractérisé en ce qu'**il consiste, en vue de la caractérisation de mouvements spécifiques donnés, à déterminer au moins une zone géographique soumise à un « appui » ou un « relâchement », ainsi qu'une séquence temporelle de sollicitation de la dite zone géographique consistant en une alternance d'une période d'activation, par appui ou relâchement, et d'une période de non activation.

2. Procédé selon la revendication 1 de détection d'un mouvement du haut du dos **caractérisé en ce que** :
• on détermine, en vue de la caractérisation du dit mouvement :
- au moins une zone géographique (D1, D2) soumise à un « appui », correspondant à une zone enveloppe des surfaces de contact avec le dossier (D) de la portion centrale du dos d'un conducteur,
- et au moins une zone géographique (F1, F2) soumise à un « relâchement », correspondant à une zone enveloppe des surfaces de contact avec l'assise (A) du fessier d'un conducteur,
• et on délivre une information de détection d'un mouvement du haut du dos, uniquement lors de la corrélation d'une zone géographique décelée avec :
- une zone géographique précitée (D1, D2) du dossier (D),
- et / ou une zone géographique précitée (F1, F2) de l'assise (A), moyennant alors, en outre, un degré minimal de symétrie, par rapport à un axe longitudinal de l'assise (A), des surfaces élémentaires constituant la dite zone géographique décelée.

3. Procédé selon la revendication 1 de détection d'un mouvement de repositionnement **caractérisé en ce que** :
• on détermine, en vue de la caractérisation du dit mouvement, deux zones géographiques (Jd, Jg) soumises à un « relâchement », correspondant chacune à une zone enveloppe de la surface de contact d'une jambe du conducteur avec l'assise (A),
• et on délivre une information de détection d'un mouvement de repositionnement, uniquement lors de la détection de la séquence temporelle suivante :
- à un instant t : corrélation d'une zone géographique décelée avec une des zones géographiques précitées (Jd, Jg) de l'assise (A),
- à un instant t+1 : absence d'activation de la zone géographique (Jd, Jg) corrélée à l'instant t, combinée avec une corrélation d'une zone géographique décelée avec l'autre zone géographique (Jg, Jd) de l'assise (A).

4. Procédé selon la revendication 1 de détection d'un mouvement de repositionnement **caractérisé en ce que** :
• on détermine, en vue de la caractérisation du dit mouvement :
- deux zones géographiques (Dbd, Dbg) soumises à un « appui », correspondant aux deux zones enveloppes respectives des surfaces de contact avec le dossier (D) des parties droite et gauche du bas du dos d'un conducteur,
- et deux zones géographiques (Pd, Pg) soumises à un « relâchement », correspondant aux deux zones enveloppes respectives des surfaces de contact avec l'assise (A) des parties droite et gauche du postérieur d'un conducteur,
• et on délivre une information de détection d'un mouvement de repositionnement lors, simultanément, de :
- la corrélation d'une zone géographique décelée avec une zone géographique précitée droite (ou gauche) (Dbd, Dbg) du dossier (D), combinée avec l'absence d'activation de l'autre zone géographique précitée (Dbg, Dbd) du dossier (D),
- la corrélation d'une zone géographique décelée avec la zone géographique précitée gauche (ou droite) (Pg, Pd) de l'assise (A), combinée avec l'absence d'activation de l'autre zone géographique précitée (Pd, Pg) de l'assise (A).

5. Procédé selon la revendication 1 de détection d'un mouvement de repositionnement **caractérisé en ce que** :
• on détermine, en vue de la caractérisation du dit mouvement :
- une zone géographique (D 3) soumise à un « appui », correspondant à la zone enveloppe de la surface de contact avec le dossier (D) du dos complet d'un conducteur,
- et une zone géographique (A3) soumise à un « relâchement », correspondant à la zone enveloppe de la surface de contact avec l'assise (A) de l'assise complète d'un conducteur,
• et on délivre une information de détection d'un mouvement de repositionnement lors de la corrélation d'une zone géographique décelée avec la zone géographique précitée (D3) du dossier (D) et / ou la zone géographique précitée (A3) de l'assise (A).

6. Procédé selon la revendication 1 de détection d'un mouvement de transition vers une position de conduite décalée, **caractérisé en ce que** :
• on détermine, en vue de la caractérisation du dit mouvement :
- deux zones géographiques (Dd, Dg) soumises à un « appui », correspondant aux deux zones enveloppes respectives des surfaces de contact avec le dossier (D) des parties droite et gauche du dos d'un conducteur,
- et deux zones géographiques (Jd, Jg) soumises à un « relâchement », correspondant chacune à une zone enveloppe de la surface de contact d'une jambe du conducteur avec l'assise (A),
• et on délivre une information de détection d'un mouvement de transition, uniquement lors de la détection de la séquence temporelle suivante :
- à un instant t : corrélation d'une zone géographique décelée avec une des zones géographiques précitées (Jd, Jg) de l'assise (A), et absence d'activation de la zone géographique (Dg, Dd) du dossier (D) symétrique de la dite zone décelée de l'assise (A),
- à un instant t+1 : absence d'activation de la zone géographique (Jd, Jg) de l'assise (A) corrélée à l'instant t, combinée avec une corrélation d'une zone géographique décelée avec l'autre zone géographique (Jg, Jd) de l'assise (A).

7. Procédé selon la revendication 1 de détection d'un mouvement de transition vers une position de conduite « enfoncée » dans le haut du dossier (D), **caractérisé en ce que** :
• on détermine, en vue de la caractérisation du dit mouvement :
- deux zones géographiques (Dh, Db) soumises à un « appui », correspondant aux deux zones enveloppes respectives des surfaces de contact avec le dossier (D) des parties haute et basse du dos d'un conducteur,
- et deux zones géographiques (Ah Ab) soumises à un « relâchement », correspondant aux deux zones enveloppes respectives des surfaces de contact avec l'assise (A) des parties haute et basse de l'assise d'un conducteur,
• et on délivre une information de détection d'un mouvement de transition lors, d'une part, de la corrélation simultanée de deux zones géographiques décelées avec respectivement les zones géographiques précitées (Dh, Ah) correspondant aux parties hautes du dos et de l'assise, et d'autre part, de l'absence combinée d'activation des zones géographiques précitées (Db, Ab) correspondant aux parties basses du dos et de l'assise.

8. Procédé de détection selon l'une des revendications précédentes **caractérisé en que** les capteurs de pression (An,n - Dn,n) consistent en des capteurs de pression différentiels aptes à mesurer la dynamique de la force appliquée sur la surface élémentaire correspondante.

9. Procédé de détection selon la revendication 8 **caractérisé en ce que**, durant une phase préalable suivant le démarrage du véhicule, dite phase de seuillage, on procède à un traitement des signaux délivrés par les capteurs de pression (An,n - Dn,n), consistant à calculer, pour chacun des dits capteurs, les moyennes respectives des plus grandes variations positives et négatives des valeurs des dits signaux, de façon à déterminer des valeurs de seuils respectivement négatif et positif pour l'extraction ultérieure des signaux.

10. Procédé de détection selon l'une des revendications précédentes **caractérisé en ce que** :
• on attribue à chaque surface élémentaire activée d'une zone géographique, un poids pondéré, par la mise en oeuvre d'une méthode mathématique telle que la logique floue, en fonction :
- de la position géographique, notamment positionnement en zone limitrophe, de la dite surface élémentaire à l'intérieur de la zone géographique,
- et / ou de l'amplitude du signal fourni par le capteur de pression associé, représentative du niveau d'activation de la dite surface élémentaire,
• et on détermine, pour chaque zone géographique, un degré d'activation de cette dernière calculé à partir du poids de chacune de ses surfaces élémentaires, de façon à associer à chaque information de détection d'un mouvement spécifique, une information représentative du degré d'appartenance à cette catégorie de mouvement.
